# EUROPEAN PATENT APPLICATION

(11) **EP 3 106 539 A1**
(43) Date of publication of application: **21.12.2016**
(21) Application number: 15749139.0
(22) Date of filing: 22.01.2015
(51) Int. Cl.: C23C 14/24, C23C 16/44

(54) **THIN FILM CLUSTER PRODUCTION SYSTEM, THIN FILM CLUSTER, THIN FILM, UV PROTECTOR, AND COSMETICS**

(30) Priority: 12.02.2014 KR 20140016013
(71) Applicant: Lee, Hyeong Gon, Seoul 151-761 (KR)
(72) Inventor: Lee, Hyeong Gon, Seoul 151-761 (KR)
(74) Representative: TBK
(86) International application number: PCT/KR2015/000665
(87) International publication number: WO 2015/122632

(57) **Abstract**

The present invention provides a thin film cluster production system characterized by producing a thin film cluster in situ and achieving improved quality, high productivity, and significantly reduced initial investment costs, a thin film cluster production method, a thin film cluster, a thin film, a UV protector, and cosmetics.

## Description

### TECHNICAL FIELD

The present disclosure relates to an apparatus for manufacturing a thin film cluster using a physical vapor deposition or chemical vapor deposition method, a thin film cluster, a thin film, an UV-blocking agent and cosmetics.

### BACKGROUND

A physical or chemical deposition method may be classified as a dry plating. The physical or chemical deposition method may be eco-friendly and thus may not cause air pollutions. For this reason, the physical or chemical deposition method may be widely used to form a thin film.

Recently, a thin film flake has been used to manufacture various products.

The thin film flake, a method for manufacturing the thin film flake, and products employing the thin film flake may be as follows: for producing a conductive paste, the thin film flake including silver (Ag), copper (Cu) or a stack of silver and copper layers may be used. Further, the thin film flake may be employed for producing a bonding paste for LED chips called as a green product, a pigment mixed with a paint and ink, cosmetics materials, sunblock particles, color particles, sintered particles, battery active material, a solar cell, a thermo-electric element, an insulating element, catalyst particles, nano-composite materials, etc.

### SUMMARY

In order to improve quality, purity and targeted properties of the thin film flake, the thin film flake is preferably produced by a dry plating using the physical or chemical deposition. However, the dry plating using the physical or chemical deposition is typically conducted in a vacuum chamber, and, hence, a production rate may be lowered and, thus, a production cost may increase. For this reason, not the dry plating but a chemical wet plating is used actually to produce the thin film flake.

When producing the thin film flake using the physical deposition in spite of a costly production, the resulting thin film flake may be highly costly. Thus, an application thereof may be limited to a very specific area.

Korean patent application publication number 2004-0068564 2004.07.31. discloses an apparatus and method for manufacturing thin film particles, which causes technical challenges which are overcome by the present disclosure. Due to the technical challenges, the approach disclosed in the above patent document may be not implemented in a real production site. That is, the approach disclosed in the above patent document may be merely a conceptional approach.

A technique for mass production of thin film particles using the physical or chemical deposition may be largely classified into following two: All two techniques may involve in-situ coating of thin film layers with large areas or of a great number thereof, and breaking thereof to form thin film clusters or thin film particles.

The two techniques may be as follows:
A first one is disclosed in US patent number 6,398,999 B1 assigned to Avery Dennison Corporation. In this approach, a great number of stack of thin film layers are formed in an in-situ manner and are used to obtain the thin film clusters or thin film particles. In order to obtain the great number of stack of thin film layers, between the thin film layer and thin film layer, a soluble or released thin film layer is formed using a deposition method. Thus, the thin film layers to be acquired and the soluble thin film layers may be alternated. Then, multi-layer thin film clusters are formed and then are unloaded out of the vacuum chamber, and, then, are first crushed. Then, in order to dissolve the soluble thin film layers, the broken multi-layer thin film clusters are inputted into a solvent or solution. Then, a second crushing is conducted to obtain thin film particles.
A second one is disclosed in US patent number 4,168,986 assigned to Polaroid Corporation or in KR patent application publication number 2004-0068564 (2004.07.31.). In this approach, before a thin film layer to be acquired is deposited, a soluble or released thin film layer is deposited on a substrate in a vacuum chamber. Then, the thin film layer to be acquired is deposited. Then, in order to separate the thin film layer from the substrate in an in-situ manner, the substrate is transferred to a separation chamber, where the deposited soluble or released thin film layer is inputted into a solution to allow the separation thereof. Then, the substrate is transferred to the deposition chamber where depositions of soluble thin film layers and target thin film layers are repeated, to obtain massive thin film particles.

The above two approaches may improve a production rate of thin film particles at some degree using the physical deposition method. However, the above two approaches may have following shortcomings which may cause the two approaches to be hardly employed in a real production site.

Regarding the first one, the target thin film layer and the soluble thin film layer are deposited alternately. Further, during a single deposition cycle, the target thin film layer and the soluble thin film layer are deposited concurrently. Thus, not only an evaporation source for the targeted thin film layer but also an evaporation source for the soluble thin film layer may be required. Further, additional devices including a power supply for supplying evaporation energy to the chamber and/or an evaporation blocking shutter coupled to the evaporation source may be required. Thus, this may lead to a complex and larger vacuum system and difficulty in an operation thereof and a process execution.

Further, in order to alternate the targeted thin film layer deposition and the soluble thin film layer deposition, the targeted thin film layer and the soluble thin film layer should be evaporated concurrently during a single cycle. The concurrent evaporation of the targeted thin film layer and the soluble thin film layer may lead to a mixing of vapors of the targeted thin film layer and the soluble thin film layer in a single vacuum chamber and thus, introduction of foreign substances in each of the targeted thin film layer and the soluble thin film layer. This may lead to deteriorations of purity and quality of each thin film layer.

Furthermore, the soluble thin film layers may be made of organic materials with high vapor pressure thereof. Thus, due to the high vapor pressure of the organic materials, the vacuum vessel, vacuum pipes and the vacuum pump system, etc. may be contaminated.

This contamination may cause the manufacturing apparatus to malfunction. Thus, the first approach may have the contamination issues for the manufacturing apparatus and the produced thin film, and thus, a deterioration of the thin film.

In the second approach, before the targeted thin film layer is deposited, the soluble thin film layer is deposited on the substrate. Then, the targeted thin film layer is deposited on the substrate. This substrate is transferred to a separation vessel to detach the thin film where the targeted thin film layer may be detached from the substrate using a predetermined solution. After detachment of the thin film layer from the substrate, the substrate is transferred to the deposition chamber. In this way, the deposition and detachment processes are repeated. In the separation vessel, massive thin film particles may be collected. In this second approach, the thin film particle production apparatus may be good in terms of a concept. However, the thin film particle production apparatus may have difficulty in practicing the same in a real production site. The manufacturing and operation of the production apparatus may be hardly achieved or may have the contamination issues for the manufacturing apparatus and the produced thin film, and thus, a deterioration of the thin film.

The production apparatus in the first and second approaches may employ a vapor deposition process of the soluble thin film layer. In order to enhance a vapor pressure of the soluble material, a heating process thereof to a high temperature may be necessary. This heating process may require a further power supply to lead to an energy consumption.

Furthermore, in order to detach the soluble thin film layer, the substrate having the thin film layer deposited thereon should be transferred to the separation vessel. Further, the substrate free of the thin film layer should be transferred to the deposition vessel. To this end, the deposition vessel and detachment vessel should share an equal vacuum atmosphere and space in spite of different vacuum levels. Thus, vapors of the solution used for detachment of the soluble thin film layer may diffuse into the deposition vessel and then act as contaminants.

In order to overcome the above problems, the present disclosure provides a manufacturing apparatus for a thin film cluster, and a thin film cluster, and a thin film produced using the manufacturing apparatus wherein massive thin film clusters are formed in an in-situ manner to improve a production rate and a product quality to avoid the above problems. An approach where the coated-material is inserted between the thin film layer and thin film layer may avoid the above problem by removing contaminant issue resulting from the high vapor pressure of the materials for obtaining the thin film cluster including a mixture of thin film layers and coated-material.

In the present disclosure, a thin film cluster manufacturing method for producing the thin film flake with high quality and ecofriendliness, a thin film cluster and a thin film produced using the method, and an UV-blocking agent and cosmetics containing the thin film flake may be supplied massively in an economical way.

A small size, and simple configuration of the vacuum apparatus used for producing the thin film cluster and thin film particle may be achieved. Further, a fabrication cost thereof and an installation space thereof may be reduced. Thus, the thin film production may be more economical.

Further, for production of the thin film cluster and thin film particle, an improved quality of the thin film and reduced contamination of the vacuum apparatus may be achieved. This may lead to an improved production rate and thus to a reduced production cost of the thin film cluster, thin film, UV-blocking agent and cosmetics with high qualities.

### BRIEF DESCRIPTION OF THE DRAWINGS

FIG. 1 is a high level diagram of a vacuum deposition chamber with a simple configuration in accordance with one embodiment of the present disclosure.
FIG. 2 is a high level diagram of a state in which a coated-material is inserted into a drum-shaped rotatable carrier with an opening at a center of a side face thereof.
FIG. 3 is a high level diagram of a state in which the drum-shaped rotatable carrier is rotated in an arrow direction and thus a coated-material film is formed on an outer face of coated-material supply means and an inner face of the carrier.
FIG. 4 is a high level diagram of a state in which the drum-shaped rotatable carrier is rotated in an arrow direction and, thus, a coated thin film layer is formed on the coated-material film and the deposition-coated thin film layer is mixed with the coated-material using collection means.
FIG. 5 is a high level diagram of a state in which a coated-material and thin film layer are coated on an outer face of the drum-shaped rotatable carrier and then are collected.
FIG. 6 is a high level diagram of one embodiment where a coated-material at a predetermined amount is received in a vessel which dispenses the coated-material downwardly and, thus, a thin film layer with a predetermined thickness is coated, and, then, the coated-material and thin film layer are moved in a predetermined direction using a movement mechanism, wherein via a switching of operation directions of the movement mechanism, supply means and collection means are changed alternatively.
FIG. 7 is a high level diagram of a manufacturing apparatus different from those as shown in FIG. 4 to FIG. 6, wherein a thin film layer is not coated on a coated-material but is coated on a substrate and then is separate from the substrate using a separation agent.
FIG. 8 is a high level diagram of a manufacturing apparatus similar to that as shown in FIG. 7 wherein a separation agent supply and collection belt is not present.
FIG. 9 is a high level diagram of a manufacturing apparatus similar to that as shown in FIG. 8 except for certain differences.
FIG. 10 is a high level diagram of a manufacturing apparatus similar to that as shown in FIG. 8 except for certain differences.
FIG. 11 is a high level diagram of a manufacturing apparatus without a collection unit wherein a separation agent and a separated thin film layer are collected via winding operation in a web form.
FIG. 12 is a high level diagram of a manufacturing apparatus where a thin film layer coated on the coated-material is collected by a continuously rotating carrier inserting the film layer into the coated-material.

### Reference numerals

1: a vacuum vessel, 3: a drum-shaped rotatable carrier with an opening at a center of a side face thereof, 5: an opening formed at a drum-shaped rotatable carrier (in one embodiment, via the opening, the coated-material and deposition source and thin film cluster may flow into and out of the drum), 7: coated-material supply and deformation means (roller or brush) (supply means 7 may act as deformation means to realize a predetermined thickness of a coated-material film), 9: collection means (roller or brush), 11: a vacuum pump system for a vacuum deposition chamber, 13: a coated-material, 15: a coated-material film resulting from flowable coated-material being applied onto an outer or inner face of the carrier via a physical force due to rotation of a drum-shaped rotatable carrier, 17: a deposition thin film layer deposited on coated-material film formed on outer or inner face of carrier, 19: deposition thin film layers mixed with the coated-material, 21: a thin film layer coating source, 23: a separation agent, 25: a coated-material film formed on an outer face of supply means in a change zone, 27: coating zone, 28: a thin film layer coated on the coated-material 13, 29: deposition thin film layers mixed with a separation agent, 37: a coated-material or separation agent supply unit, 38: a movement mechanism, 39: a collection unit, 53: a drum-shaped rotatable carrier (on an outer face of which, a coated-material and thin film layer are coated), 55: a coated-material film coated on an outer face of the carrier, 57: a thin film layer coated on the coated-material film 55, 59: separation knife or means, 101: a substrate, 103: a thin film layer formed on the substrate, 105 : a separation agent film formed by separation agent supply and deformation means, 107: a separation agent supply and collection belt, 109: piping means to return a separation agent and/or thin film layer from the collection unit to the supply unit 37, 115: a coated-material collected having inserted at least two thin film layers therein at a predetermined point, 119: thin film layer insertion means, 177: separation agent supply and deformation means, 201: a winding auxiliary roller, 211: collection in a web form with a small outer diameter just after being wound on the substrate and auxiliary roller 201 after the separation agent bonded to the thin film layer is separated from the substrate, 213: collection in a web form with a large outer diameter in a considerable time after being wound on the substrate and auxiliary roller 201 after the separation agent bonded to the thin film layer is separated from the substrate.

### DETAILED DESCRIPTIONS

In one aspect of the present disclosure, an apparatus for manufacturing a thin film cluster including a stack of at least two thin film layers sandwiching a coated-material therebetween is provided, wherein the apparatus comprises:
a vacuum vessel having a vacuum atmosphere space defined therein in which the thin film cluster is formed;
vacuum pumping means to realize a vacuum state at least in the space;
at least one coating source to coat a thin film of a predetermined substance on a predetermined surface in the vacuum vessel, wherein the thin film includes at least one layer;
a supply unit to supply at least the coated-material to a predetermined location;
a collection unit to collect at least the coated-material; and
a coating zone in which the thin film is coated on a predetermined surface of the coated-material using the at least one coating source;
wherein the coated-material is present in the supply unit and collection unit and coating zone at least for a predetermined period,
wherein the coated-material is different at least between the supply unit and collection unit in terms of at least one of a thickness, a structure, a shape, a phase, and a movement form thereof,
wherein the coated-material is guided to move from the supply unit at least to the collection unit,
wherein in the coating zone, the thin film layer is at least partially coated on the coated-material surface,
wherein after coating, the coated-material is collected into a predetermined location including at least the collection unit,
wherein at least at a predetermined timing,
the supply unit, coating zone, collection unit are arranged in this order such that the coated-material moves at least from the supply unit, to the coating zone, and then to the collection unit,
wherein in the coating zone, at least a portion of the coated-material having the thin film including the coating source substance coated thereon is supplied from the supply unit,
wherein at least a portion of the coated-material collected into the collection unit is supplied from the supply unit, and passes through the coating zone and then is collected together with at least a portion of the thin film,
wherein at least one location between the supply unit and the collection unit, a coated-material change zone is provided,
wherein in the change zone, at least one of a thickness, structure, shape, phase, and movement form of the coated-material is changed,
wherein the coated-material is changed at least one time in the change zone and then is collected,
wherein a maximum length of the thin film in a coated state on the coated-material is at least two times larger than the thin film thickness,
wherein at least a portion of the coated-material is supplied from the supply unit to the coating zone in an in-situ manner with a predetermined viscosity level to enable a flowability of the coated-material without being deposited in a vapor state,
wherein the thickness of the thin film is between 0.1 nanometer and 50 microns,
wherein at least a portion of the coated-material is collected into the collection unit in an in-situ manner with a predetermined viscosity level to enable a flowability of the coated-material without being deposited in a vapor state,
wherein a maximum area of the thin film in a coated state on the coated-material is at least two times larger than a maximum area of the thin film in the collection unit,
wherein the coated-material includes at least one of a flowable substance, a room temperature flowable substance, and a plastic substance,
wherein a saturated vapor pressure of the coated-material at 25° C is below 100 torr,
wherein a melting point of the coated-material is below 650° C,
wherein after the thin film is coated on the coated-material surface, the thin film is guided to move at least to the collection unit, and collections in the collection unit include at least the coated-material and the thin film layer, wherein at least at a predetermined timing, a stack of the at least two thin film layers sandwiching the coated-material therebetween is acquired.

As used herein, a term "thin film layer" may be exchangeable with a term "thin film". However, the former may be used to give an emphasis on a layered shape of the thin film. A term "thin film particle" may be exchangeable with a term "thin film". However, the former may be used to give an emphasis on a particulate shape of the thin film.

The coated-material may not be evaporated via a heating. Nevertheless, the coated-material may preferably have a lower saturated vapor pressure in a room temperature state at least in the vacuum apparatus. The coated-material may preferably have a saturated vapor pressure below 100 torr in a room temperature state at least in the vacuum apparatus. When the coated-material has a saturated vapor pressure above 100 torr, vapors of the coated-material may interfere a thin film layer formation and/or act as a contaminant, which may be different based on a structure and property of the physical or chemical deposition apparatus.

The coated-material should be, in an in-situ manner, deformed and/or moved via a physical force at least for a predetermined period. For this reason, the coated-material may employ a flowable substance and/or plastic substance. Further, it may be preferable that the coated-material may be softened in a deformable and/or flowable state and/or may have a lower melting temperature.

However, in order to enlarge a selectin of the coated-material substance, it may be preferable that the coated-material has the melting point below 650°C. When the coated-material has the melting point above 650°C, it may require a further energy to reach the melting temperature and the deposition apparatus may have a structure and material enduring the high temperature.

The coated-material in an in-situ state may require absence of a solvent. Most of solvents may have a very high vapor pressure to act as a contaminant into the vacuum atmosphere in the vacuum apparatus and has an adverse effect on the vacuum pump and other parts in the apparatus. Thus, the coated-material may be transitioned and moved without involving a solvent, and thus, may be collected with the thin film layers.

In another aspect of the present disclosure, an apparatus for manufacturing a thin film cluster including a coated-material and a thin film layer winding at least ten times the coated-material is provided, wherein the apparatus comprises:
a vacuum vessel having a vacuum atmosphere space defined therein in which the thin film cluster is formed;
vacuum pumping means to realize a vacuum state at least in the space;
at least one coating source to coat a thin film of a predetermined substance on a predetermined surface in the vacuum vessel, wherein the thin film includes at least one layer;
a supply unit to supply at least the coated-material to a predetermined location;
a collection unit to collect at least the coated-material in a web form;
a coating zone in which the thin film is coated on a predetermined surface of the coated-material using the at least one coating source; and
a power device to supply a power to enable a movement and/or deformation of at least the coated-material,
wherein the coated-material is present in the supply unit and collection unit and coating zone at least for a predetermined period,
wherein the coated-material is different at least between the supply unit and collection unit in terms of at least one of a thickness, a structure, a shape, a phase, and a movement form thereof,
wherein the coated-material is guided to move from the supply unit at least to the collection unit,
wherein in the coating zone, the thin film layer is at least partially coated on the coated-material surface,
wherein after coating, the coated-material is collected into a predetermined location including at least the collection unit,
wherein at least at a predetermined timing,
the supply unit, coating zone, collection unit are arranged in this order such that the coated-material moves at least from the supply unit, to the coating zone, and then to the collection unit,
wherein in the coating zone, at least a portion of the coated-material having the thin film including the coating source substance coated thereon is supplied from the supply unit,
wherein at least a portion of the coated-material collected into the collection unit is supplied from the supply unit, and passes through the coating zone and then is collected in the collection unit in a web form,
wherein at least one location between the supply unit and the collection unit, a coated-material change zone is provided,
wherein in the change zone, at least one of a thickness, structure, shape, phase, and movement form of the coated-material is changed,
wherein the coated-material is changed at least one time in the change zone and then is collected,
wherein a maximum length of the thin film in a coated state on the coated-material is at least two times larger than the thin film thickness,
wherein at least a portion of the coated-material is supplied from the supply unit to the coating zone in an in-situ manner with a predetermined viscosity level to enable a flowability of the coated-material without being deposited in a vapor state,
wherein the thickness of the thin film is between 0.1 nanometer and 50 microns,
wherein at least a portion of the coated-material is collected into the collection unit in an in-situ manner via winding operation in a web form without involving a solvent and without being deposited in a vapor state,
wherein the coated-material includes at least one of a flowable substance, a room temperature flowable substance, and a plastic substance,
wherein a saturated vapor pressure of the coated-material at 25° C is below 100 torr,
wherein a melting point of the coated-material is below 650° C,
wherein after the thin film is coated on the coated-material surface, the thin film is guided to move at least to the collection unit, and collections in the collection unit include at least the coated-material and the thin film layer, wherein at least at a predetermined timing, the thin film cluster including the coated-material and the thin film layer winding at least ten times the coated-material is acquired.

This aspect may be different from the above-described aspect in that the thin film cluster including the coated-material and the thin film layer winding at least ten times the coated-material is acquired in the collection unit.

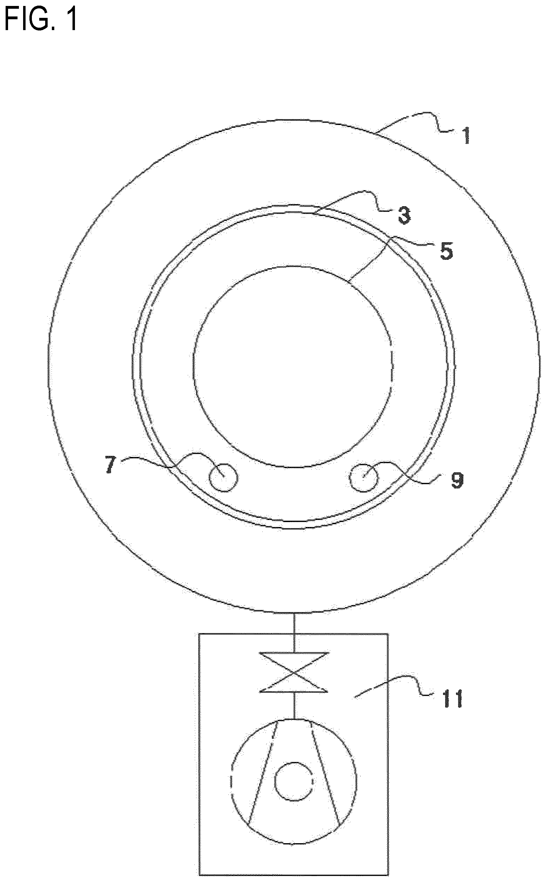

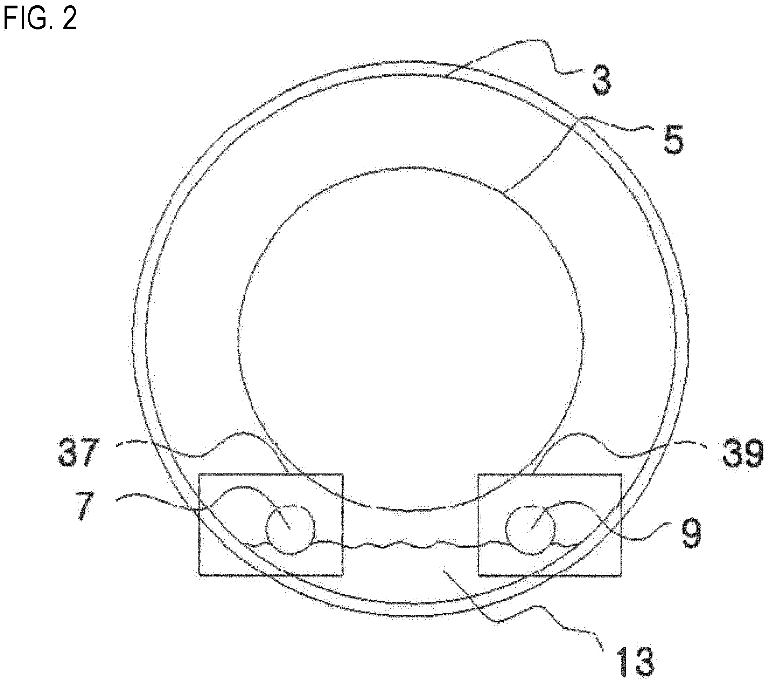

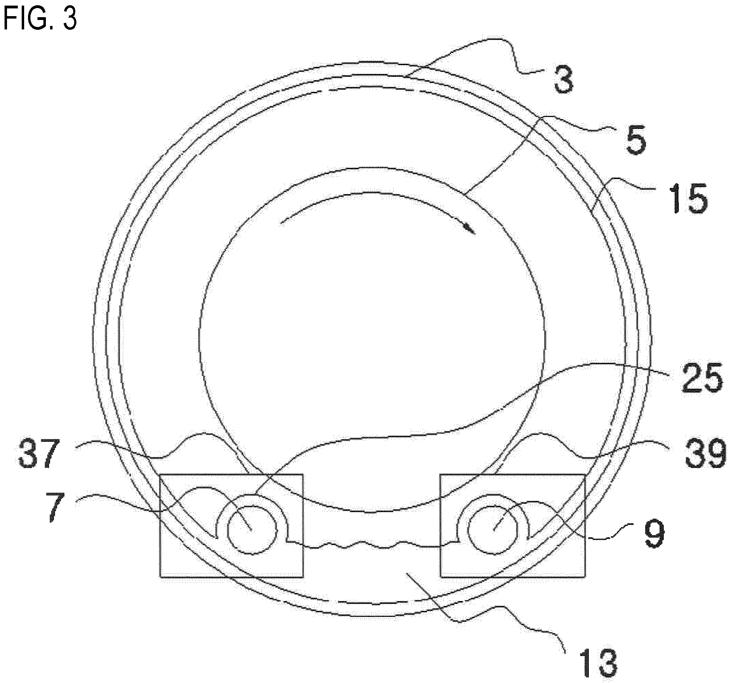

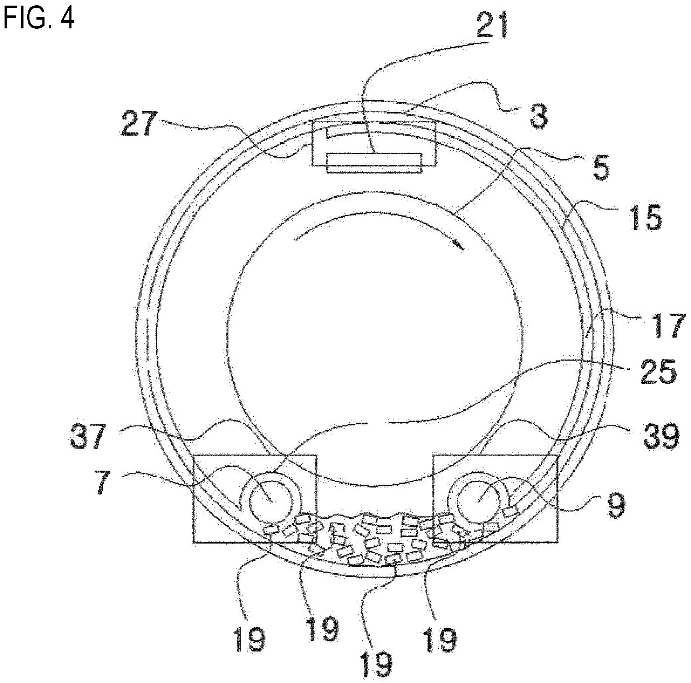

Among the manufacturing apparatus of the present disclosure, the manufacturing apparatus associated with the coated-material may be described, by way of example, with reference to FIG. 1 to FIG. 6.

The manufacturing apparatus in accordance with one aspect of the present disclosure may include a vacuum deposition chamber 1; a vacuum pump system 11; a drum-shaped rotatable carrier 3 having an opening 5 which, by way of example, is formed at a center of a side face of the carrier; coated-material supply and/or deformation means 7 provided in an axial direction of the carrier 3; coated-material collection means 9; and a power device (not shown) coupled to the rotatable carrier 3. Within the carrier 3, the coated-material 13 and thin film layer coating source 21 may be disposed. An operation of the manufacturing apparatus may be as follows:

Components required to form a thin film cluster may be loaded into the vacuum deposition chamber in an in-situ manner. An inner space of the vacuum deposition chamber 1 may be pumped using the vacuum pump system 11 to meet a desired vacuum level.

Then, the power device may be activated to rotate the rotatable carrier 3. The coated-material 13 gathered on a bottom of the carrier 3 may form a coated-material film 15 over a portion of an inner face of the carrier via a rotation of the carrier 3.

On the coated-material film 15, a thin film layer 17 may be coated using the thin film layer coating source 21.

The coated-material film 15 and thin film layer 17 may rotate into the coated-material 13 gathered on a bottom of the carrier 3. A further rotation may allow the coated-material film 15 and thin film layer 17 to move toward the coating source 21. In this connection, based on the coated-material film 15 thickness or viscosity or carrier rotation speed, etc. the thin film layer including the coated-material film invaded into the coated-material 13 may be partially collected into the coated-material 13 gathered on a bottom of the carrier 3, thereby to collect the thin film cluster as a product of the manufacturing apparatus of the present disclosure. Of course, due to various factors, while the collected thin film layer may be partially contained in the coated-material film 15, the collected thin film layer may be partially moved to the coating source to be coated as a further thin film layer with the coated-material thin film being interposed therebetween. Although this process may be achieved without the coated-material supply and deformation means 7 and collection means 9, the coated-material supply and deformation means 7 and collection means 9 may be introduced if necessary. The coated-material supply and deformation means 7 and collection means 9 may be formed of a blade, roll, brush, knife, etc. by way of example. The coated-material supply and deformation means 7 and collection means 9 may be operatively coupled to the power device.

Further, when the coated-material is plastic or flowing material, a heater may be required to heat the coated-material to a predetermined temperature. However, the present disclosure is not limited thereto. When the coated-material is flowing at a room temperature, it may dispense with the heater. In the same way, whether to introduce the heater may be applied to a separation material used in another aspect of the present disclosure.

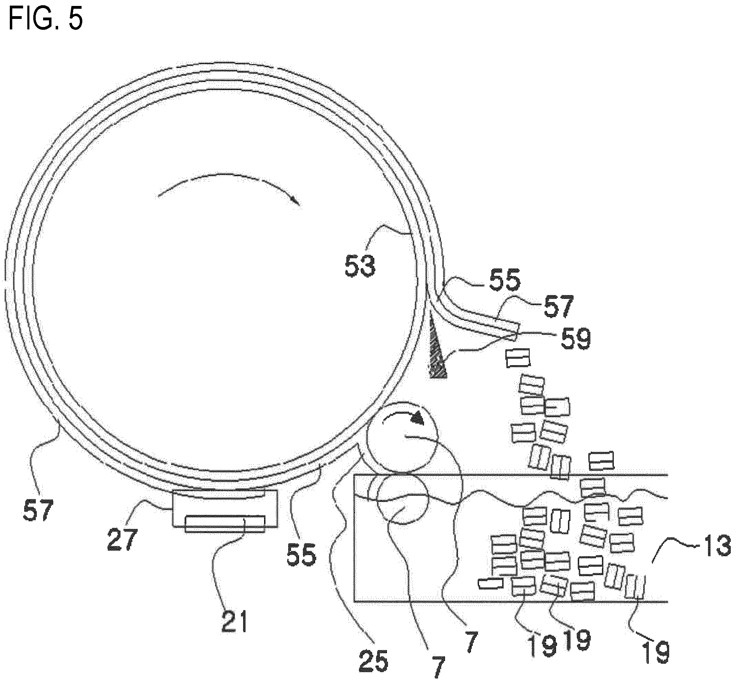

Another aspect of the present disclosure, that is, a manufacturing apparatus as shown in FIG. 5 may be similar to the thin film cluster manufacturing apparatus in accordance with the above aspect of the present disclosure except that the coated-material film 55 is coated on an outer face of the carrier, and supply means 7 for forming the coated-material film and a separation knife 59 are employed.

In another aspect of the present disclosure,
an apparatus for manufacturing a thin film cluster including a stack of at least two thin film layers sandwiching a coated-material therebetween is provided, wherein the apparatus comprises:
a vacuum vessel having a vacuum atmosphere space defined therein in which the thin film cluster is formed;
vacuum pumping means to realize a vacuum state at least in the space;
at least one coating source to coat a thin film of a predetermined substance on a predetermined surface in the vacuum vessel, wherein the thin film includes at least one layer;
a coating zone in which the thin film is coated on a predetermined surface of the coated-material using the at least one coating source; and
thin film layer insertion means to insert the thin film layer coated on the coated-material surface into the coated-material continuously or intermittently,
wherein the coated-material with a thickness equal to or larger than a predetermined thickness is disposed in a carrier or vessel or is disposed in a free-standing mode,
wherein the thin film cluster is collected by inserting the thin film layer into the coated-material,
wherein at least at a predetermined timing,
a maximum length of the thin film in a coated state on the coated-material is at least two times larger than the thin film thickness,
the coated-material coated in the coating zone has at least one thin film layer inserted therein,
the thin film layer insertion means collects the thin film layer into the coated-material by inserting the thin film layer coated on the coated-material surface into the coated-material intermittently or continuously for a predetermined period,
wherein the thickness of the thin film is between 0.1 nanometer and 50 microns,
wherein at least a portion of the coated-material in the coating zone moves in a predetermined mode in an in-situ manner with a predetermined viscosity level to enable a flowability of the coated-material without being deposited in a vapor state,
wherein a maximum area of the thin film in a coated state on the coated-material is at least two times larger than a maximum area of the thin film collected in the coated-material,
wherein the coated-material includes:
   at least one of a flowable substance, a room temperature flowable substance, and a plastic substance,
   wherein a saturated vapor pressure of the coated-material at 25° C is below 100 torr,
   wherein a melting point of the coated-material is below 650° C,
   wherein the thin film in collections collected in the coated-material is acquired as a stack of the at least two thin film layers sandwiching the coated-material therebetween.

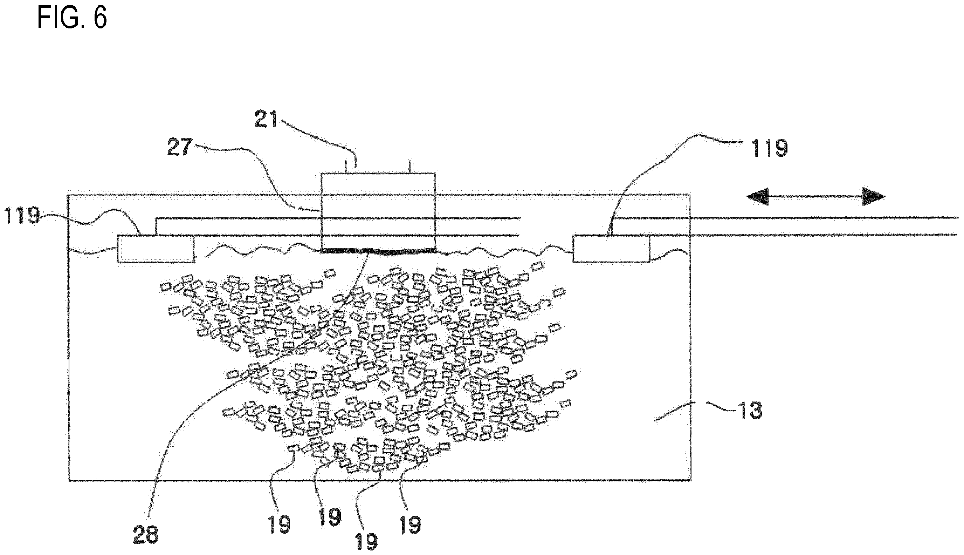

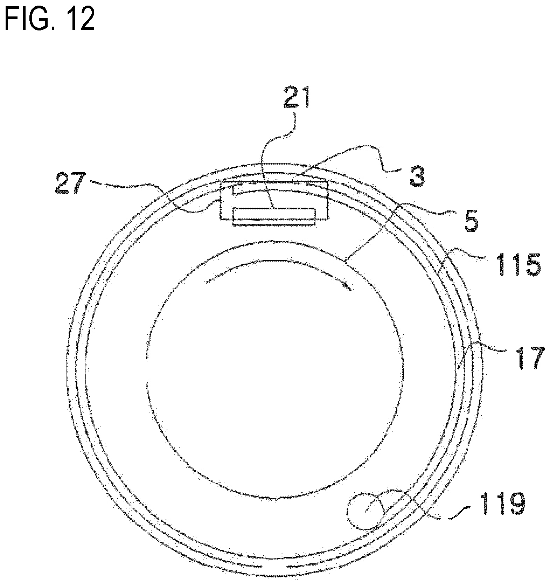

The manufacturing apparatus in accordance with this aspect of the present disclosure may be configured as shown in FIG. 6 or FIG. 12. The manufacturing apparatus may include a vessel containing a coated-material 13, thin film layer insertion means 119, and a coating source 21. A thin film layer 28 may be coated on a surface of the coated-material 13 using the coating source 21 to reach a predetermined thickness. Then, the thin film layer insertion means 119 may be displaced from a current location to an opposite location thereto to allow the thin film layer 28 to be inserted into the coated-material 13 and to be collected. When the thin film layer 28 is coated on the coated-material 13 again to reach the predetermined thickness, the thin film layer insertion means 119 may be displaced from a current location to an opposite location thereto to allow the thin film layer 28 to be inserted into the coated-material 13 and to be collected. This process may be repeated. This approach may allow the manufacturing apparatus with a simple configuration. However, the thin film layer 28 may be discontinuous. However, this discontinuity may be suitable for producing the thin film cluster. The manufacturing apparatus with this configuration may be illustrated in FIG. 6 or FIG. 12. Compared to the manufacturing apparatus as shown in FIG. 6 where the insertion means 119 may be displaced intermittently, a manufacturing apparatus as shown in FIG. 12 may have a continuously-rotating vessel or carrier to receive therein a coated-material where a continuously-coated thin film layer is continuously inserted into the coated-material and thus is collected into the coated-material. In this manufacturing apparatus, the supply means and collection mean may be integrated into a single unit.

The coated-material may be at least partially disposed in the carrier or vessel. In an alternative, the coated-material may be thermal plastic or thermal flowable substance to have a predetermined shape in a free-standing mode. The latter coated-material may be collected by inserting the thin film layer into the free standing coated-material using predetermined thin film layer.

The insertion of the thin film layer may be embodied as a pressure-insertion of the thin film layer. The present disclosure is not limited thereto. The insertion of the thin film layer may be embodied by moving the coated-material above the thin film layer. In this connection, the coated-material moved above the thin film layer may be a coated-material disposed beneath the thin film layer or supplied from another separate supply unit by way of example. Further, in addition to the mechanical insertion of the thin film layer, the thin film layer may be inserted via spraying of the coated-material. In this connection, when employing the spraying of the coated-material, a spraying pump (not shown) act as the thin film layer insertion means.

In one example, a thin film cluster with more various forms or more complex specifications may be required. In this connection, in another aspect of the present disclosure, an apparatus for manufacturing a thin film cluster including at least two thin film layers sandwiching a separation agent therebetween is provided, wherein the apparatus comprises:
a vacuum vessel having a vacuum atmosphere space defined therein in which the thin film cluster is formed;
vacuum pumping means to realize a vacuum state at least in the space;
at least one coating source to coat a thin film of a predetermined substance on a predetermined surface in the vacuum vessel, wherein the thin film includes at least one layer;
a substrate to provide a coating surface on which the thin film is to be coated, wherein the substrate is disposed around the coating source;
a separation agent supply unit to supply the separation agent on the predetermined surface of the thin film and/or substrate;
a separation agent collection unit to collect at least the separation agent;
a coating zone in which the thin film is coated on a predetermined surface of the substrate using the at least one coating source;
a bonding zone in which the separation agent and thin film and substrate are bonded to each other; and
a separation zone in which the separation agent together with the thin film is separated from the substrate;
wherein at least for a predetermined period, the separation agent is present in the supply unit and bonding zone and collection unit, wherein the separation agent is different at least between the supply unit and collection unit in terms of at least one of a thickness, a structure, a shape, a phase, and a movement form thereof,
wherein a separation agent change zone is present in at least one location between the supply unit and bonding zone and collection unit,
and,
wherein the separation agent moves from the supply unit at least to the collection unit, wherein, after the separation agent is bonded to the thin film coated on the substrate, the separation agent together with the thin film are separated from the substrate and thus are collected into the predetermined location including at least the collection unit,
wherein, at least at a predetermined timing,
the bonding zone extend from a first point at which the separation agent is bonded to the thin film coated on the substrate to a second point at which the separation agent is separated from the substrate,
wherein the separation agent is used to separate the thin film from the substrate,
wherein in the change zone, at least one of a thickness, structure, shape, and phase of the separation agent is changed,
wherein a maximum length of the thin film in a coated state on the substrate is at least two times larger than the thin film thickness,
wherein at least a portion of the separation agent supplied from the supply unit and then bonded to the thin film and then, separated from the substrate is collected into the collection unit,
wherein at least a portion of the separation agent is supplied to be bonded to the thin film in an in-situ manner with a predetermined viscosity level to enable a flowability of the separation agent without being deposited in a vapor state,
wherein the thickness of the thin film is between 0.1 nanometer and 50 microns,
wherein at least a portion of the separation agent is collected into the collection unit in an in-situ manner without involving a solvent and without being deposited in a vapor state,
wherein the supply unit, the bonding zone, the separation zone, and the collection unit are arranged in this order in terms of a movement of the separation agent,
a maximum area of the thin film in a coated state on the substrate is at least two times larger than a maximum area of the thin film in the collection unit,
wherein the separation agent includes at least one of a flowable substance, a room temperature flowable substance, and a plastic substance, wherein a saturated vapor pressure of the coated-material at 25° C is below 100 torr, wherein a melting point of the coated-material is below 650° C,
wherein after the thin film layer is coated on the substrate, the thin film layer moves at least via the bonding zone, and separation zone, and to the collection unit, wherein the collection collected into the collection unit includes at least the separation agent and the thin film layer, wherein at least at a predetermined timing, at least two thin film layers sandwiching the separation agent therebetween are collected.

This approach where the thin film is coated on the substrate and then the thin film layer is separated from the substrate using the separation agent may be different from the above aspect where the thin film is coated on the coated-material.

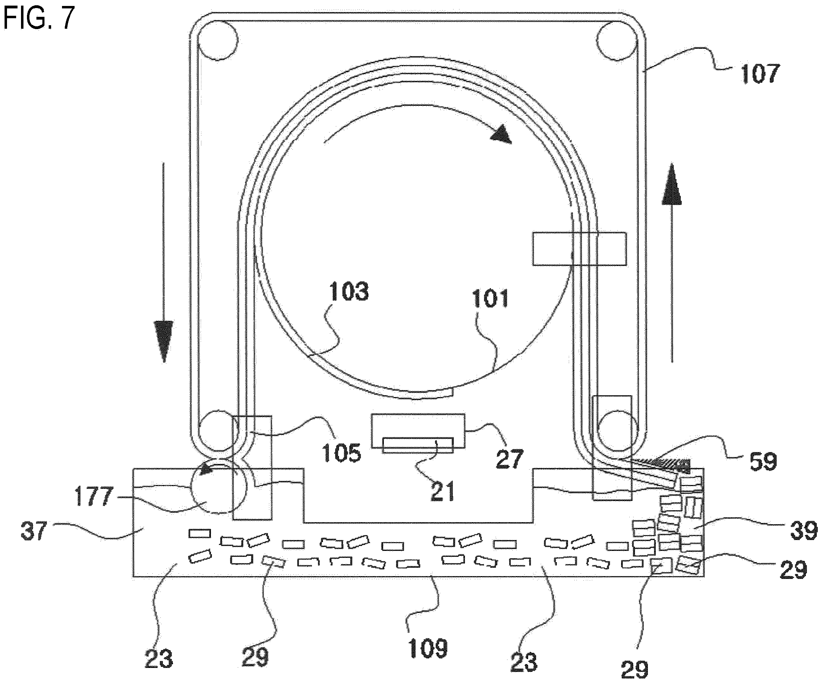

The manufacturing apparatus in accordance with this aspect will be described with reference to FIG. 7 by way of example.

First, not the coated-material 13 but the substrate 101 is prepared. The thin film layer 103 may be coated not on the coated-material 13 but on the substrate 101 on at least one surface thereof. The thin film layer deposited on the substrate surface may have a very small thickness, and the substrate surface may be made of a solid. Thus, using a knife or brush, the thin film layer may not be easily detached from the substrate surface. For this reason, in the present disclosure, the separation agent 23 with an adhesive force above a predetermined level may be introduced. The separation agent 23 may be bonded onto the surface of the thin film layer 103 deposited on the substrate surface. Then, the separation agent and bonded thin film layer are detached from the substrate. This bonding and detachment may be repeated. In this way, the separation agent and thin film layer may be collected. Thus, the manufacturing apparatus of the present disclosure may produce the thin film cluster. The separation agent may be flowable or flexible during being bonded at least to the thin film layer. The approach in this aspect may be applied without a limitation. By way of example, a deposition condition for the thin film layer may include a heating of the substrate, or applying a bias voltage to the substrate. This may be useful when collecting a thin film layer with micro patterns or three-dimensional patterns or elongate wire patterns, or when producing micro thin film flakes regularized or standardized into a predetermined shape. By way of example, thin film layers may be produced for manufacturing a sliver wire, graphene, batter active material, UV-blocking agent, electromagnetic blocking agent or absorption agent, pearl pigment, etc.

When the separation agent 23 is bonded to the thin film layer and the thin film layer bonded to the separation agent is detached from the substrate, the separation agent deposition is not used but means for apply the physical force to the separation agent is used. The means for apply the physical force to the separation agent to bond the separation agent to the thin film layer or detach the thin film from the substrate may include, by way of example, a blade, knife, brush, belt, roller, etc.

In another aspect of the present disclosure, an apparatus for manufacturing a thin film cluster including a separation agent and a thin film layer winding at least ten times the separation agent is provided, wherein the apparatus comprises:
a vacuum vessel having a vacuum atmosphere space defined therein in which the thin film cluster is formed;
vacuum pumping means to realize a vacuum state at least in the space;
at least one coating source to coat a thin film of a predetermined substance on a predetermined surface in the vacuum
a substrate to provide a coating surface on which the thin film is to be coated, wherein the substrate is disposed around the coating source;
a separation agent supply unit to supply the separation agent on the predetermined surface of the thin film and/or substrate;
a separation agent collection unit to collect at least the separation agent;
a coating zone in which the thin film is coated on a predetermined surface of the substrate using the at least one coating source;
a bonding zone in which the separation agent and thin film and substrate are bonded to each other; and
a separation zone in which the separation agent together with the thin film is separated from the substrate;
wherein at least for a predetermined period, the separation agent is present in the supply unit and bonding zone and collection unit, wherein the separation agent is different at least between the supply unit and collection unit in terms of at least one of a thickness, a structure, a shape, a phase, and a movement form thereof,
wherein a separation agent change zone is present in at least one location between the supply unit and bonding zone and collection unit,
wherein the separation agent moves from the supply unit at least to the collection unit, wherein, after the separation agent is bonded to the thin film coated on the substrate, the separation agent together with the thin film are separated from the substrate and thus are collected into the predetermined location including at least the collection unit,
wherein, at least at a predetermined timing, the bonding zone extend from a first point at which the separation agent is bonded to the thin film coated on the substrate to a second point at which the separation agent is separated from the substrate,
wherein the separation agent is used to separate the thin film from the substrate,
wherein in the change zone, at least one of a thickness, structure, shape, and phase of the separation agent is changed,
wherein a maximum length of the thin film in a coated state on the substrate is at least two times larger than the thin film thickness,
wherein at least a portion of the separation agent supplied from the supply unit and then bonded to the thin film and then, separated from the substrate is collected into the collection unit,
wherein at least a portion of the separation agent is supplied to be bonded to the thin film in an in-situ manner with a predetermined viscosity level to enable a flowability of the separation agent without being deposited in a vapor state,
wherein the thickness of the thin film is between 0.1 nanometer and 50 microns,
wherein at least a portion of the separation agent is collected into the collection unit in an in-situ manner in a web form without involving a solvent and without being deposited in a vapor state,
wherein the supply unit, the bonding zone, the separation zone, and the collection unit are arranged in this order in terms of a movement of the separation agent,
wherein after the thin film layer is coated on the substrate, the thin film layer moves at least via the bonding zone, and separation zone, and to the collection unit, wherein the collection collected into the collection unit includes at least the separation agent and the thin film layer, wherein at least at a predetermined timing, the thin film cluster including the separation agent and the thin film layer winding at least ten times the separation agent is collected.

In this aspect, the substrate may be completely different from the coated-material. By way of example, the substrate surface may be made of a substance with a lower adhesive force to the thin film or the substrate surface may be subjected to a surface treatment to form an easily released surface. This may allow the thin film layer to be more easily detached from the substrate surface.

The thin film layer deposited on the substrate surface may have a very small thickness, and the substrate surface may be made of a solid. Thus, using a knife or brush, the thin film layer may not be easily detached from the substrate surface. For this reason, in the present disclosure, the separation agent 9 with an adhesive force above a predetermined level may be introduced. The separation agent 9 may be bonded onto the surface of the thin film layer 1 deposited on the substrate surface. Then, the separation agent and bonded thin film layer are detached from the substrate. This bonding and detachment may be repeated. In this way, the separation agent and thin film layer may be collected. Thus, the manufacturing apparatus of the present disclosure may produce the thin film cluster.

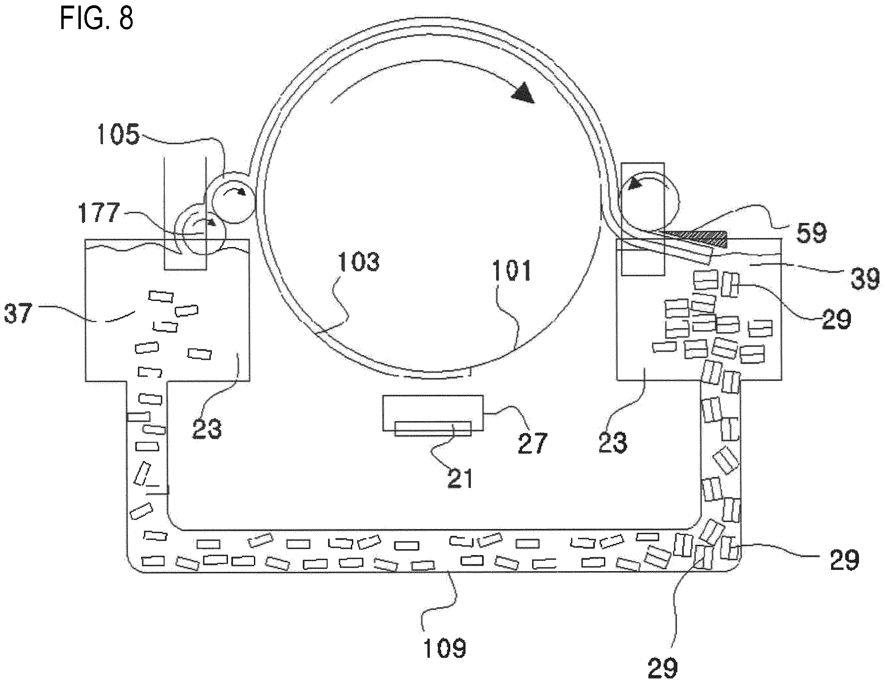

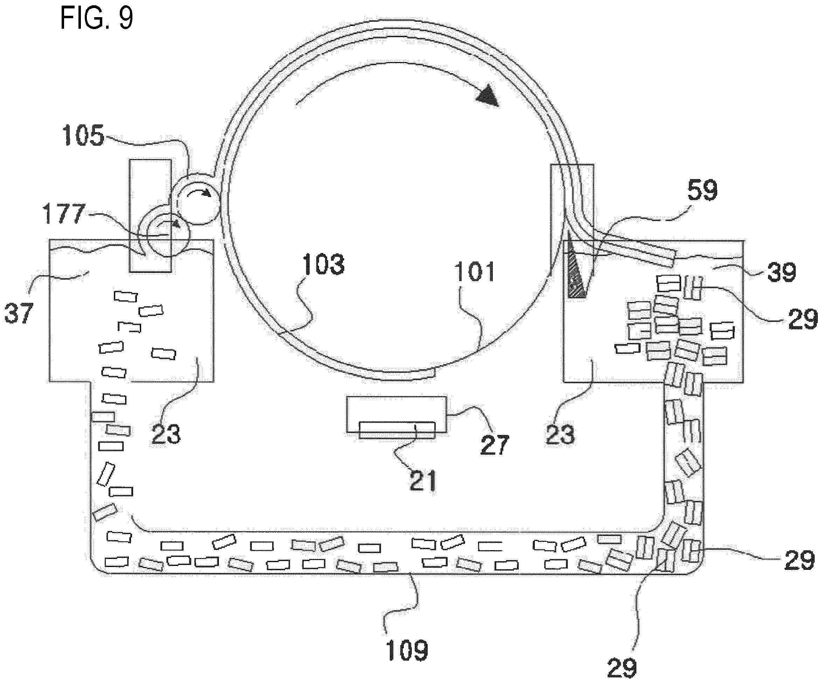

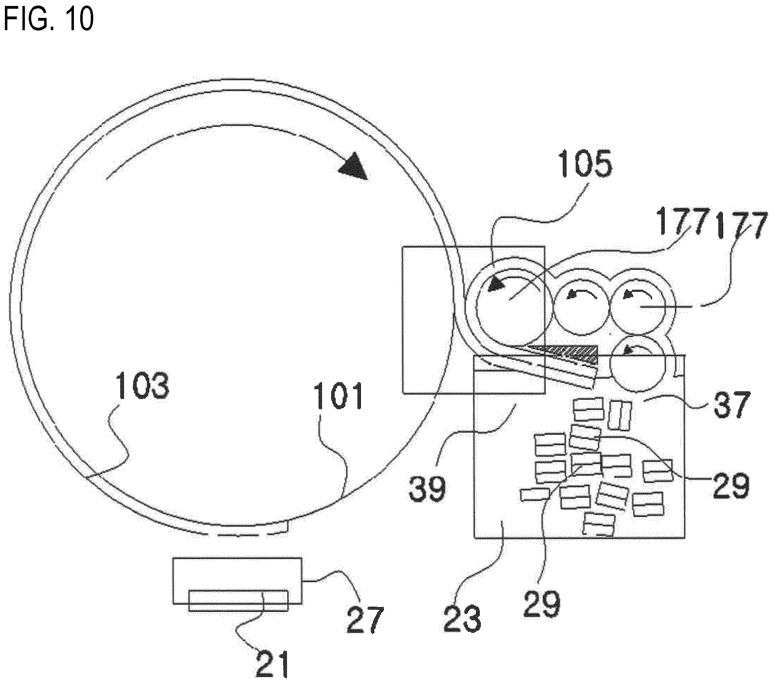

The manufacturing apparatus employing the separation agent and substrate as in this aspect of the present disclosure is shown in FIG. 7 to FIG. 11. In FIG. 7 to FIG. 9, a separation agent supply unit 37 and a collection unit 39 are connected to each other via piping means 109. In FIG. 10, the separation agent supply unit 37 and collection unit 39 are integrated into a single vessel containing therein the separation agent.

An operation of the manufacturing apparatus employing the separation agent and substrate may involve the coating of the thin film layer 103 on a surface of the substrate 101 using the coating source 21 in the vacuum vessel pumped to a predetermined vacuum level. This may be similar to that in above aspects.

Onto the thin film layer 103, a separation agent film 105 may be bonded or attached.

The thin film layer 103 bonded to the separation agent film 105 may be detached from the substrate 101.

The separated thin film layer 103 and separation agent film 105 are collected into the collection unit. In this way, the manufacturing apparatus of the present disclosure may produce the thin film cluster.

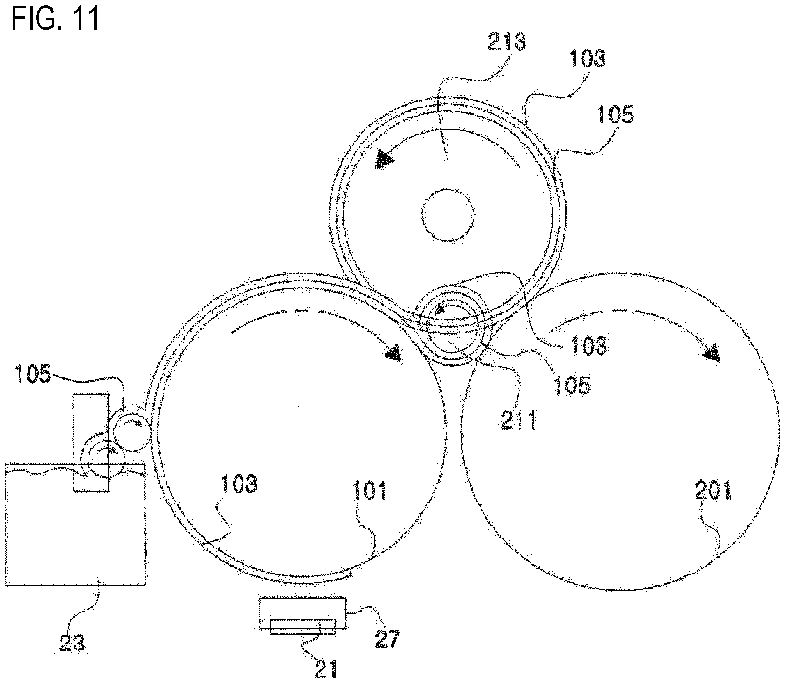

In this kind of aspects, especially, as shown in FIG. 11, the collected separation agent and thin film layer may be collected in a wound web state.

In the present disclosure, the supply unit, change zone, bonding zone, separation zone, and collection unit may be individual. However, at least two of the supply unit, change zone, bonding zone, separation zone, and collection unit may be integrated.

The separation agent may not be evaporated via a heating. The separation agent may preferably have a lower saturated vapor pressure in a room temperature state at least in the vacuum apparatus. The separation agent may preferably have a saturated vapor pressure below 100 torr in a room temperature state at least in the vacuum apparatus. When the separation agent has a saturated vapor pressure above 100 torr, vapors of the separation agent may interfere a thin film layer formation and/or act as a contaminant, which may be different based on a structure and property of the physical or chemical deposition apparatus.

The separation agent should be, in an in-situ manner, deformed and/or moved via a physical force at least for a predetermined period. For this reason, the separation agent may employ a flowable substance and/or plastic substance. Further, it may be preferable that the separation agent may be softened in a deformable and/or flowable state and/or may have a lower melting temperature.

However, in order to enlarge a selectin of the separation agent substance, it may be preferable that the separation agent has the melting point below 650°C. When the separation agent has the melting point above 650°C, it may require a further energy to reach the melting temperature and the deposition apparatus may have a structure and material enduring the high temperature.

The separation agent mixed, in an in-situ manner, with the thin film layer may require absence of a solvent. Most of solvents may have a very high vapor pressure to act as a contaminant into the vacuum atmosphere in the vacuum apparatus and has an adverse effect on the vacuum pump and other parts in the apparatus. Thus, the separation agent may be transitioned and moved without involving a solvent, and thus, may be collected with the thin film layers.

By way of example, using the process of this aspect, a method for manufacturing the thin film cluster in accordance with the present disclosure may be achieved. However, the collection process may not be limited to the above embodiment. For example, the gravity may allow natural collection. Further, prior to a collection via the gravity, collection means such as a blade, knife, brush, etc. to apply the physical force to display the coated-material or separation agent including the thin film layer may be employed for compulsive collections. However, the collection process may not be limited thereto.

Thus, in the present disclosure, the coated-material and separation agent may be formed by a non-deposition method.

Further, vapors of coating sources resulting from heated deposition sources for the coated-material deposition and/or radiation heats from the deposition source, as described above may be removed. Thus, the detrimental factors may not affect the vacuum apparatus. The thin film product may have good quality and economical cost.

Furthermore, in addition to this, the solvent may not be involved in the collection process due to an in-situ collection. This may lead to further advantages.

Furthermore, a physical property and structure of the coated-material and/or separation agent included in the thin film cluster manufacturing apparatus of the present disclosure may allow continuous productions in an in-situ manner which are not possible from the conventional thin film cluster manufacturing apparatus. Thus, this may lead to improved production rate and economy of the resultant thin film cluster.

During the separation agent is bonded at least to the thin film layer, the separation agent may be preferably flowable or flexible. However, the present disclosure is not limited thereto. By way of example, for deposition of the thin film layer, the substrate may be heated. Further, for deposition of the thin film layer in micro pattern or fine three dimensional patterns, for production of the thin film particle well-defined into a predetermined shape, the heating may be useful.

In order to bond the separation agent 9 to the thin film layer and/or detach the separation agent and the thin film layer from the substrate, the separation agent deposition may not be employed, but a physical force may be applied to the separation agent. To this end, physical force-application means may be used for the bonding and detachment. Examples of the physical force-application means may include a blade, knife, brush, belt, roller, etc.

At least one of the collection process of at least portion of the coated-material or separation agent together with the thin film layers via the physical force, and a movement process to the coating zone should be conducted in an in-situ manner.

The thin film cluster produced by the manufacturing apparatus of the present disclosure may include at least two thin film layers and the coated-material or separation agent interposed there between.

By way of example, the vacuum vessel may be a vacuum deposition chamber. The present disclosure is not limited thereto. The vacuum vessel may perform the physical vapor deposition such as a sputtering or the chemical vapor deposition.

Forms of the coated-material may not be limited to a specific condition or shape. The coated-material may have at least one surface on which the predetermined thin film layer may be formed using physical or chemical deposition.

The thin film cluster or thin film particle produced by the thin film cluster manufacturing apparatus may have a single layer structure or a stack of multi-layers made of different substances. The thin film cluster or thin film particle may be three-dimensional. The thin film cluster or thin film particle may be employed as cosmetics ingredients, pigments, paints, medical material, electronic materials, catalyst particles, battery active substance, e-ink printing material, et. In addition, a thin film cluster may be very useful of which the thin film has at least one layer having an electrical conductivity or thermal conductivity greater than an electrical conductivity or thermal conductivity of another layer therein. This thin film cluster may be applied as a wide variety of electrical connecting means, for example, for mounting, wiring, connecting, bonding, soldering, welding, brazing, sintering, etc. of printed electronics, and electronic electricity units. This provides a variety of benefits, such as reduction of the amount of the noble metals.

The thin film cluster or thin film particle may provide the UV-blocking agent with various effects. The thin film cluster or thin film particle may be useful to produce the UV-blocking agent mainly made of inorganic materials. The UV-blocking agent of the present disclosure may include at least one different substance in addition to the main UV-blocking substance.

In the thin film cluster or thin film particle produced by the manufacturing apparatus of the present disclosure, the coated-material may be made of a flowable substance or plastic substance. At a predetermined timing and/or for a predetermined period, at least a portion of the thin film layers may be separated from each other without involving a separate solution. As described above, the coated-material may employ the flowable substance or plastic substance. The coated-material may have a saturated vapor pressure sufficiently low not to suppress the formation of the thin film via physical deposition.

A ratio of a length to a thickness the thin film coated on the coated-material or separation agent as collected is smaller than 2, not a thin film form but a micro particle form may be achieved. This may not produce the thin film particle in a flake form as a target product of the present disclosure.

Further, since the thin film produced by a wet thin film plating method is not eco-friendly and has a lower purity, it may be preferable that the thin film layer is formed using a physical deposition method. The thin film deposition may be conformal to the coated-material surface shape in order to maintain the thin film form.

In order to produce massive thin film layers with a predetermined thickness, the coated-material or separation agent separating adjacent thin film layers should be added thereon in an in-situ manner. The addition of the coated-material or separation agent should not involve the vapor deposition. This is because the vapor deposition of the coated-material or separation agent to separate the adjacent thin film layers therebetween may lead to mutual diffusion and mixing of vapors from the coated-material or separation agent. This diffusion may act as contamination sources. Thus, the purity and quality of the thin film may be deteriorated. Further, as described above, the vacuum apparatus and accompanying parts may be polluted. For this reason, the addition of the coated-material or separation agent should not involve the vapor deposition

Thus, in order that the coated-material or separation agent separating the adjacent thin film layers is added thereon in an in-situ manner without involving the vapor deposition, the present coated-material or separation agent may include the flowable substance or plastic substance. Thus, the flowable substance or plastic substance may be interposed between the adjacent thin film layers. When the coated-material or separation agent includes the flowable substance, in particular, a room temperature flowable substance, the heating process may be not required. This may lead to a simple process.

However, when the coated-material is made of the flowable substance, a fine shape may be non-available. Thus, in order that the thin film layer has a three-dimensional shape or a predetermined shape, a thermal flowable substance or thermal plastic substance may be used. Using the thermal flowable substance or thermal plastic substance, an imprinting method may be used. In this connection, using a heated mold, a thin film of a three-dimensional shape to be acquired may be imprinted on the coated-material surface. In this way, the thin film with the three-dimensional shape corresponding to that formed in the mold may be produced continuously.

In this way, using the flowable coated-material or separation agent, it may dispense with an evaporation process, and, thus, the vapor deposition. Rather, between the thin film layers, the coated-material or separation agent may be added via the physical force. The above-described mutual diffusion and thus contaminations may be removed.

When the thin film layer thickness is below 0.1 nanometer, the thin film layer form may not be maintained. When the thin film layer thickness is above 50 microns, the thin film layer may not be crushed into a fine thin film, and, further, the excessive material consumption may occur.

In still another aspect of the present disclosure,
at least a portion of the thin film includes a stack of at least two layers made of different substances. For example, a chemical stability of at least one layer in the thin film may be larger than that of another layer therein. Further, a human body-related safety or stimulus of at least one layer in the thin film may be larger than that of another layer therein.

In still another aspect of the present disclosure,
when the thin film layer is coated on the coated-material or substrate, the surface of the coated-material or substrate has at least partially a recessed and/or protruded portion into a three-dimensional predetermined shape, wherein at least a portion of the thin film has a structure conformal to the three-dimensional predetermined shape. In this connection, the thin film layer may be easily formed into the thin film particle standardized into a predetermined shape. By way of example, the thin film layer may be formed into a three-dimensional shape and then, may be sliced into a two-dimensional shape or substantially a two-dimensional shape during producing standardized thin film particles in an in-situ or unladed state.

In still another aspect of the present disclosure,
wherein the coated-material or separation agent is supplied to at least a portion of a predetermined carrier or vessel, and the carrier or vessel includes a film-type, roll-type, or circulation-type belt, drum or vessel.

In still another aspect of the present disclosure, it may be preferable that a number of the thin film layers sandwiching the coated-material or separation agent to form the thin film cluster is as large as possible. In this way, per a single vacuum vessel, a production rate of the thin film cluster may be enhanced.

In the present disclosure, in the thin film cluster or thin film particle, the coated-material may be made of a flowable substance or plastic substance, and, hence, at a predetermined timing and/or for a predetermined period, at least a portion of the thin film layers may be separated from each other and thus may be displaced. As described above, the coated-material may include the flowable substance or plastic substance. The saturated vapor pressure thereof may be lower enough not to suppress the thin film via the physical deposition.

Further, since the thin film produced by a wet thin film plating method is not eco-friendly and has a lower purity, it may be preferable that the thin film layer is formed using a deposition method. The thin film deposition may be conformal to the coated-material surface shape in order to maintain the thin film form.

In order to produce massive thin film layers with a predetermined thickness, the coated-material or separation agent separating adjacent thin film layers should be added thereon in an in-situ manner. The addition of the coated-material or separation agent should not involve the vapor deposition. This is because the vapor deposition of the coated-material or separation agent to separate the adjacent thin film layers therebetween may lead to mutual diffusion and mixing of vapors from the coated-material or separation agent. This diffusion may act as contamination sources. Thus, the purity and quality of the thin film may be deteriorated. Further, as described above, the vacuum apparatus and accompanying parts may be polluted. For this reason, the addition of the coated-material or separation agent should not involve the vapor deposition

Thus, in order that the coated-material or separation agent separating the adjacent thin film layers is added thereon in an in-situ manner without involving the vapor deposition, the present coated-material or separation agent may include the flowable substance or plastic substance. Thus, the flowable substance or plastic substance may be interposed between the adjacent thin film layers. When the coated-material or separation agent includes the flowable substance, in particular, a room temperature flowable substance, the heating process may be not required. This may lead to a simple process.

However, when the coated-material or separation agent is made of the flowable substance, a fine shape may be non-available. Thus, in order that the thin film layer has a three-dimensional shape or a predetermined shape, a thermal flowable substance or thermal plastic substance may be used. Using the thermal flowable substance or thermal plastic substance, an imprinting method may be used. In this connection, using a heated mold, a thin film of a three-dimensional shape to be acquired may be imprinted on the coated-material surface. In this way, the thin film with the three-dimensional shape corresponding to that formed in the mold may be produced continuously.

In this way, using the flowable coated-material or separation agent, it may dispense with an evaporation process, and, thus, the vapor deposition. Rather, between the thin film layers, the coated-material or separation agent may be added via the physical force. The above-described mutual diffusion and thus contaminations may be removed.

The coated-material or separation agent may not be evaporated via a heating. Nevertheless, the coated-material or separation agent may preferably have a lower saturated vapor pressure in a room temperature state at least in the vacuum apparatus. The coated-material or separation agent may preferably have a saturated vapor pressure below 100 torr in a room temperature state at least in the vacuum apparatus. When the coated-material or separation agent has a saturated vapor pressure above 100 torr, vapors of the coated-material or separation agent may interfere a thin film layer formation and/or act as a contaminant, which may be different based on a structure and property of the physical or chemical deposition apparatus.

The coated-material or separation agent should be, in an in-situ manner, deformed and/or moved via a physical force at least for a predetermined period. For this reason, the coated-material or separation agent may employ a flowable substance and/or plastic substance. Further, it may be preferable that the coated-material or separation agent may be softened in a deformable and/or flowable state and/or may have a lower melting temperature.

However, in order to enlarge a selectin of the coated-material substance or separation agent, it may be preferable that the coated-material or separation agent has the melting point below 650°C. When the coated-material or separation agent has the melting point above 650°C, it may require a further energy to reach the melting temperature and the deposition apparatus may have a structure and material enduring the high temperature.

The coated-material or separation agent in an in-situ state may require absence of a solvent. Most of solvents may have a very high vapor pressure to act as a contaminant into the vacuum atmosphere in the vacuum apparatus and has an adverse effect on the vacuum pump and other parts in the apparatus. Thus, the coated-material or separation agent may be transitioned and moved without involving a solvent, and thus, may be collected with the thin film layers.

By way of example, for the production of the thin film cluster, at least a portion of the thin film layers resulting from the deposition process may be crushed to be collected. The size of the crushed piece of the thin film layer may be smaller than 1/100 of the thin film layer resulting from the deposition process, that is, a non-crushed thin film layer. At least a portion of the crushing process may be concurrent, in an in-situ manner, with the addition of the coated-material or separation agent to the thin film. However, the present disclosure is not limited thereto. If necessary, the thin film may be withdrawn out of the vacuum vessel, and then may be put into a separate crushing and screening apparatus. In one example embodiment, in an in-situ manner, using a motor-driven rotatable brush, the coated-material and thin film layer may be subjected to the mixing and crushing operations via physical forces of the brush. As a result, in the thin film layer deposition, the thin film layer in a sheet shape may have an area of about 1 m². Then, the area size of the crushed piece of the thin film layer may have 1 to 100 micron*micron. For this, at least a portion of the motor-driven rotatable brush may be installed to at least partially overlap the coated-material and thin film layer. Since the coated-material is made of the flowable substance or plastic substance, using a mixing unit such as the brush, it may dispense with the solvent and, thus, the thin film layers and coated-material may be mixed sufficiently via the physical force. Further, by the mixing process, new coated-material substances may be moved or supplied onto one or more thin film layer or onto a deposition region facing a thin film layer deposition source, that is, the coating zone.

When the thin film layer thickness is below 0.1 nanometer, the thin film layer form may not be maintained. When the thin film layer thickness is above 50 microns, the thin film layer may not be crushed into a fine thin film, and, further, the excessive material consumption may occur. By way of example, the produced thin film cluster may be crushed into thin film particles. The crushed thin film particle may have an area smaller than 1/100 than that of the thin film cluster deposited in an in-situ state or an unloaded state out of the vacuum vessel.

Further, in the present disclosure, the coated-material may employ a mixture of at least two substances in order to adjust properties of the coated-material including a viscosity, saturated vapor pressure, melting point.

Key components of the manufacturing apparatus of the present disclosure have been described, by way of example, for the skilled person to the art to understand the structures of the manufacturing apparatus. Additional parts and/or associated processes to implement the manufacturing apparatus may be well known to the skilled person to the art. Further, the components of the manufacturing apparatus of the present disclosure as described above may have modifications or alternations well known to the skilled person to the art from the above-defined teachings of the present disclosure.

In still another aspect of the present disclosure, the coating source may be plural, and at least one coating source of the plural coating sources may form a thin film layer of a different substance from that from at least another coating source, and the thin film may include at least two different substances. In this aspect, the thin film may have a multi-layer structure or a gradation structure or an alloy or mixture or hybrid structure. Example of the thin film with the structures may include, by way of example, an UV-blocking thin film including a protective layer and/or color-rendering layer coated on either or both of faces of the UV-blocking thin film; an UV-blocking thin film including a gradation structure wherein the concentration of the color-rendering layer and/or protective layer may gradually increase toward the surface of the thin film, etc. Further, the thin film with another structure may include an electronic material thin film having a copper thin film and a silver and/or gold thin film coated on either or both of faces of the copper thin film; or a gradation structure with a copper thin film having a silver and/or gold thin film coated on either or both of faces thereof wherein the concentration of the silver and/or gold may gradually increase toward the surface of the copper film, etc.

In still another aspect of the present disclosure, a thin film cluster produced by the thin film cluster manufacturing apparatus as above defined may be provided.

In still another aspect of the present disclosure, a thin film may be produced from the thin film cluster as above defined, wherein at least a portion of the coated-material or separation agent is removed from the thin film cluster to produce the thin film.

In still another aspect of the present disclosure, the thin film as above defined includes a primary functional material M and at least one secondary functional material S.

In still another aspect of the present disclosure, the primary functional material M has electric-conductive, UV-blocking, thermal-conductive, photo-electric, thermo-electric, catalyst, anti-corrosion, reflective, insulating, welding, sintering, and/or coating functions.

In still another aspect of the present disclosure, the thin film has a multi-layer structure, gradation structure, and/or hybrid structure.

In still another aspect of the present disclosure, the thin film includes an UV-blocking substance film.

In still another aspect of the present disclosure, there is provided an UV-blocking thin film including the thin film as defined above, wherein the thin film includes a primary UV-blocking substance A made of an inorganic UV-blocking substance, and at least one secondary functional material S.

In still another aspect of the present disclosure, the secondary functional material S has a UV-blocking level lower than that of the primary UV-blocking substance A and includes at least one color-rendering substance rendering a predetermined color, wherein at least a portion of the color-rendering substance is disposed on the surface of the thin film to be exposed outsides. Alternatively, the secondary functional material S may be protective layer, or the secondary functional material S may have a protective layer thereon, or an additional layer may be interposed between the primary UV-blocking layer A and secondary functional material S.

In still another aspect of the present disclosure, the UV-blocking thin film as defined above is provided wherein a color of at least one color-rendering substance in the secondary functional material S is at least one selected from a yellow, red, and black, wherein the UV-blocking thin film has a predetermined color-rendering property without an additional color-rendering material added thereto to remove or reduce a white cloudiness degree.

In still another aspect of the present disclosure, an UV-blocking agent may be provided having the thin film as defined above.

In still another aspect of the present disclosure, cosmetics may be provided having the thin film as defined above.

The present disclosure may have various applications as described above. Especially, when the thin film employs a copper, the present disclosure may have countless applications.

## Claims

1. an apparatus for manufacturing a thin film cluster including a stack of at least two thin film layers sandwiching a coated-material therebetween is provided, wherein the apparatus comprises:
a vacuum vessel having a vacuum atmosphere space defined therein in which the thin film cluster is formed;
vacuum pumping means to realize a vacuum state at least in the space;
at least one coating source to coat a thin film of a predetermined substance on a predetermined surface in the vacuum vessel, wherein the thin film includes at least one layer;
a supply unit to supply at least the coated-material to a predetermined location;
a collection unit to collect at least the coated-material; and
a coating zone in which the thin film is coated on a predetermined surface of the coated-material using the at least one coating source;
wherein the coated-material is present in the supply unit and collection unit and coating zone at least for a predetermined period,
wherein the coated-material is different at least between the supply unit and collection unit in terms of at least one of a thickness, a structure, a shape, a phase, and a movement form thereof,
wherein the coated-material is guided to move from the supply unit at least to the collection unit,
wherein in the coating zone, the thin film layer is at least partially coated on the coated-material surface,
wherein after coating, the coated-material is collected into a predetermined location including at least the collection unit,
wherein at least at a predetermined timing,
the supply unit, coating zone, collection unit are arranged in this order such that the coated-material moves at least from the supply unit, to the coating zone, and then to the collection unit,
wherein in the coating zone, at least a portion of the coated-material having the thin film including the coating source substance coated thereon is supplied from the supply unit,
wherein at least a portion of the coated-material collected into the collection unit is supplied from the supply unit, and passes through the coating zone and then is collected together with at least a portion of the thin film,
wherein at least one location between the supply unit and the collection unit, a coated-material change zone is provided,
wherein in the change zone, at least one of a thickness, structure, shape, phase, and movement form of the coated-material is changed,
wherein the coated-material is changed at least one time in the change zone and then is collected,
wherein a maximum length of the thin film in a coated state on the coated-material is at least two times larger than the thin film thickness,
wherein at least a portion of the coated-material is supplied from the supply unit to the coating zone in an in-situ manner with a predetermined viscosity level to enable a flowability of the coated-material without being deposited in a vapor state,
wherein the thickness of the thin film is between 0.1 nanometer and 50 microns,
wherein at least a portion of the coated-material is collected into the collection unit in an in-situ manner with a predetermined viscosity level to enable a flowability of the coated-material without being deposited in a vapor state,
wherein a maximum area of the thin film in a coated state on the coated-material is at least two times larger than a maximum area of the thin film in the collection unit,
wherein the coated-material includes at least one of a flowable substance, a room temperature flowable substance, and a plastic substance,
wherein a saturated vapor pressure of the coated-material at 25° C is below 100 torr,
wherein a melting point of the coated-material is below 650° C,
wherein after the thin film is coated on the coated-material surface, the thin film is guided to move at least to the collection unit, and collections in the collection unit include at least the coated-material and the thin film layer, wherein at least at a predetermined timing, a stack of the at least two thin film layers sandwiching the coated-material therebetween is acquired.

2. An apparatus for manufacturing a thin film cluster including a coated-material and a thin film layer winding at least ten times the coated-material is provided, wherein the apparatus comprises:
a vacuum vessel having a vacuum atmosphere space defined therein in which the thin film cluster is formed;
vacuum pumping means to realize a vacuum state at least in the space;
at least one coating source to coat a thin film of a predetermined substance on a predetermined surface in the vacuum vessel, wherein the thin film includes at least one layer;
a supply unit to supply at least the coated-material to a predetermined location;
a collection unit to collect at least the coated-material in a web form;
a coating zone in which the thin film is coated on a predetermined surface of the coated-material using the at least one coating source; and
a power device to supply a power to enable a movement and/or deformation of at least the coated-material,
wherein the coated-material is present in the supply unit and collection unit and coating zone at least for a predetermined period,
wherein the coated-material is different at least between the supply unit and collection unit in terms of at least one of a thickness, a structure, a shape, a phase, and a movement form thereof,
wherein the coated-material is guided to move from the supply unit at least to the collection unit,
wherein in the coating zone, the thin film layer is at least partially coated on the coated-material surface,
wherein after coating, the coated-material is collected into a predetermined location including at least the collection unit,
wherein at least at a predetermined timing,
the supply unit, coating zone, collection unit are arranged in this order such that the coated-material moves at least from the supply unit, to the coating zone, and then to the collection unit,
wherein in the coating zone, at least a portion of the coated-material having the thin film including the coating source substance coated thereon is supplied from the supply unit,
wherein at least a portion of the coated-material collected into the collection unit is supplied from the supply unit, and passes through the coating zone and then is collected in the collection unit in a web form,
wherein at least one location between the supply unit and the collection unit, a coated-material change zone is provided,
wherein in the change zone, at least one of a thickness, structure, shape, phase, and movement form of the coated-material is changed,
wherein the coated-material is changed at least one time in the change zone and then is collected,
wherein a maximum length of the thin film in a coated state on the coated-material is at least two times larger than the thin film thickness,
wherein at least a portion of the coated-material is supplied from the supply unit to the coating zone in an in-situ manner with a predetermined viscosity level to enable a flowability of the coated-material without being deposited in a vapor state,
wherein the thickness of the thin film is between 0.1 nanometer and 50 microns,
wherein at least a portion of the coated-material is collected into the collection unit in an in-situ manner via winding operation in a web form without involving a solvent and without being deposited in a vapor state,
wherein the coated-material includes at least one of a flowable substance, a room temperature flowable substance, and a plastic substance,
wherein a saturated vapor pressure of the coated-material at 25° C is below 100 torr,
wherein a melting point of the coated-material is below 650° C,
wherein after the thin film is coated on the coated-material surface, the thin film is guided to move at least to the collection unit, and collections in the collection unit include at least the coated-material and the thin film layer, wherein at least at a predetermined timing, the thin film cluster including the coated-material and the thin film layer winding at least ten times the coated-material is acquired.

3. An apparatus for manufacturing a thin film cluster including a stack of at least two thin film layers sandwiching a coated-material therebetween is provided, wherein the apparatus comprises:
a vacuum vessel having a vacuum atmosphere space defined therein in which the thin film cluster is formed;
vacuum pumping means to realize a vacuum state at least in the space;
at least one coating source to coat a thin film of a predetermined substance on a predetermined surface in the vacuum vessel, wherein the thin film includes at least one layer;
a coating zone in which the thin film is coated on a predetermined surface of the coated-material using the at least one coating source; and
thin film layer insertion means to insert the thin film layer coated on the coated-material surface into the coated-material continuously or intermittently,
wherein the coated-material with a thickness equal to or larger than a predetermined thickness is disposed in a carrier or vessel or is disposed in a free-standing mode,
wherein the thin film cluster is collected by inserting the thin film layer into the coated-material,
wherein at least at a predetermined timing,
a maximum length of the thin film in a coated state on the coated-material is at least two times larger than the thin film thickness,
the coated-material coated in the coating zone has at least one thin film layer inserted therein,
the thin film layer insertion means collects the thin film layer into the coated-material by inserting the thin film layer coated on the coated-material surface into the coated-material intermittently or continuously for a predetermined period,
wherein the thickness of the thin film is between 0.1 nanometer and 50 microns,
wherein at least a portion of the coated-material in the coating zone moves in a predetermined mode in an in-situ manner with a predetermined viscosity level to enable a flowability of the coated-material without being deposited in a vapor state,
wherein a maximum area of the thin film in a coated state on the coated-material is at least two times larger than a maximum area of the thin film collected in the coated-material,
wherein the coated-material includes:
at least one of a flowable substance, a room temperature flowable substance, and a plastic substance,
wherein a saturated vapor pressure of the coated-material at 25° C is below 100 torr,
wherein a melting point of the coated-material is below 650° C,
wherein the thin film in collections collected in the coated-material is acquired as a stack of the at least two thin film layers sandwiching the coated-material therebetween.

4. An apparatus for manufacturing a thin film cluster including at least two thin film layers sandwiching a separation agent therebetween is provided, wherein the apparatus comprises:
a vacuum vessel having a vacuum atmosphere space defined therein in which the thin film cluster is formed;
vacuum pumping means to realize a vacuum state at least in the space;
at least one coating source to coat a thin film of a predetermined substance on a predetermined surface in the vacuum vessel, wherein the thin film includes at least one layer;
a substrate to provide a coating surface on which the thin film is to be coated, wherein the substrate is disposed around the coating source;
a separation agent supply unit to supply the separation agent on the predetermined surface of the thin film and/or substrate;
a separation agent collection unit to collect at least the separation agent;
a coating zone in which the thin film is coated on a predetermined surface of the substrate using the at least one coating source;
a bonding zone in which the separation agent and thin film and substrate are bonded to each other; and
a separation zone in which the separation agent together with the thin film is separated from the substrate;
wherein at least for a predetermined period, the separation agent is present in the supply unit and bonding zone and collection unit, wherein the separation agent is different at least between the supply unit and collection unit in terms of at least one of a thickness, a structure, a shape, a phase, and a movement form thereof,
wherein a separation agent change zone is present in at least one location between the supply unit and bonding zone and collection unit,
wherein the separation agent moves from the supply unit at least to the collection unit, wherein, after the separation agent is bonded to the thin film coated on the substrate, the separation agent together with the thin film are separated from the substrate and thus are collected into the predetermined location including at least the collection unit,
wherein, at least at a predetermined timing,
the bonding zone extend from a first point at which the separation agent is bonded to the thin film coated on the substrate to a second point at which the separation agent is separated from the substrate,
wherein the separation agent is used to separate the thin film from the substrate,
wherein in the change zone, at least one of a thickness, structure, shape, and phase of the separation agent is changed,
wherein a maximum length of the thin film in a coated state on the substrate is at least two times larger than the thin film thickness,
wherein at least a portion of the separation agent supplied from the supply unit and then bonded to the thin film and then, separated from the substrate is collected into the collection unit,
wherein at least a portion of the separation agent is supplied to be bonded to the thin film in an in-situ manner with a predetermined viscosity level to enable a flowability of the separation agent without being deposited in a vapor state,
wherein the thickness of the thin film is between 0.1 nanometer and 50 microns,
wherein at least a portion of the separation agent is collected into the collection unit in an in-situ manner without involving a solvent and without being deposited in a vapor state,
wherein the supply unit, the bonding zone, the separation zone, and the collection unit are arranged in this order in terms of a movement of the separation agent,
wherein the separation agent includes at least one of a flowable substance, a room temperature flowable substance, and a plastic substance, wherein a saturated vapor pressure of the coated-material at 25° C is below 100 torr, wherein a melting point of the coated-material is below 650° C,
wherein the collection collected into the collection unit includes at least the separation agent and the thin film layer, wherein at least at a predetermined timing, at least two thin film layers sandwiching the separation agent therebetween are collected.

5. An apparatus for manufacturing a thin film cluster including a separation agent and a thin film layer winding at least ten times the separation agent is provided, wherein the apparatus comprises:
a vacuum vessel having a vacuum atmosphere space defined therein in which the thin film cluster is formed;
vacuum pumping means to realize a vacuum state at least in the space;
at least one coating source to coat a thin film of a predetermined substance on a predetermined surface in the vacuum
a substrate to provide a coating surface on which the thin film is to be coated, wherein the substrate is disposed around the coating source;
a separation agent supply unit to supply the separation agent on the predetermined surface of the thin film and/or substrate;
a separation agent collection unit to collect at least the separation agent;
a coating zone in which the thin film is coated on a predetermined surface of the substrate using the at least one coating source;
a bonding zone in which the separation agent and thin film and substrate are bonded to each other; and
a separation zone in which the separation agent together with the thin film is separated from the substrate;
wherein at least for a predetermined period, the separation agent is present in the supply unit and bonding zone and collection unit, wherein the separation agent is different at least between the supply unit and collection unit in terms of at least one of a thickness, a structure, a shape, a phase, and a movement form thereof,
wherein a separation agent change zone is present in at least one location between the supply unit and bonding zone and collection unit,
wherein the separation agent moves from the supply unit at least to the collection unit, wherein, after the separation agent is bonded to the thin film coated on the substrate, the separation agent together with the thin film are separated from the substrate and thus are collected into the predetermined location including at least the collection unit,
wherein, at least at a predetermined timing, the bonding zone extend from a first point at which the separation agent is bonded to the thin film coated on the substrate to a second point at which the separation agent is separated from the substrate,
wherein the separation agent is used to separate the thin film from the substrate,
wherein in the change zone, at least one of a thickness, structure, shape, and phase of the separation agent is changed,
wherein a maximum length of the thin film in a coated state on the substrate is at least two times larger than the thin film thickness,
wherein at least a portion of the separation agent supplied from the supply unit and then bonded to the thin film and then, separated from the substrate is collected into the collection unit,
wherein at least a portion of the separation agent is supplied to be bonded to the thin film in an in-situ manner with a predetermined viscosity level to enable a flowability of the separation agent without being deposited in a vapor state,
wherein the thickness of the thin film is between 0.1 nanometer and 50 microns,
wherein at least a portion of the separation agent is collected into the collection unit in an in-situ manner in a web form without involving a solvent and without being deposited in a vapor state,
wherein the supply unit, the bonding zone, the separation zone, and the collection unit are arranged in this order in terms of a movement of the separation agent,
wherein after the thin film layer is coated on the substrate, the thin film layer moves at least via the bonding zone, and separation zone, and to the collection unit, wherein the collection collected into the collection unit includes at least the separation agent and the thin film layer, wherein at least at a predetermined timing, the thin film cluster including the separation agent and the thin film layer winding at least ten times the separation agent is collected.

6. The apparatus of one of claims 1 to 5, wherein at least a portion of the thin film includes a stack of at least two layers made of different su bsta nces.

7. The apparatus of one of claims 1 to 5, wherein when the thin film layer is coated on the coated-material or substrate, the surface of the coated-material or substrate has at least partially a recessed and/or protruded portion into a three-dimensional predetermined shape, wherein at least a portion of the thin film has a structure conformal to the three-dimensional predetermined shape.

8. The apparatus of one of claims 1 to 5, wherein the coated-material or separation agent is supplied to at least a portion of a predetermined carrier or vessel, and the carrier or vessel includes a film-type, roll-type, or circulation-type belt, drum or vessel.

9. The apparatus of one of claims 1 to 5, wherein the coating source is plural, and at least one coating source of the plural coating sources forms a thin film layer of a different substance from that from at least another coating source, and the thin film includes at least two different substances.

10. A thin film cluster produced by the apparatus of one of claims 1 to 5.

11. A thin film produced from the thin film cluster of claim 10, wherein at least a portion of the coated-material or separation agent is removed from the thin film cluster to produce the thin film.

12. The thin film of claim 11, wherein the thin film includes a primary functional material M and at least one secondary functional material S.

13. The thin film of claim 12, wherein the primary functional material M has electric-conductive, UV-blocking, thermal-conductive, photo-electric, thermo-electric, catalyst, anti-corrosion, reflective, insulating, welding, sintering, and/or coating functions.

14. The thin film of claim 11, wherein the thin film has a multi-layer structure, gradation structure, and/or hybrid structure.

15. The thin film of claim 11, wherein the thin film includes an UV-blocking substance film.

16. An UV-blocking thin film including the thin film of claim 11, wherein the thin film includes a primary UV-blocking substance A made of an inorganic UV-blocking substance, and at least one secondary functional material S.

17. The UV-blocking thin film of claim 16, wherein the secondary functional material S has a UV-blocking level lower than that of the primary UV-blocking substance A and includes at least one color-rendering substance rendering a predetermined color, wherein at least a portion of the color-rendering substance is disposed on the surface of the thin film to be exposed outsides.

18. The UV-blocking thin film of claim 16, wherein a color of at least one color-rendering substance in the secondary functional material S is at least one selected from a yellow, red, and black, wherein the UV-blocking thin film has a predetermined color-rendering property without an additional color-rendering material added thereto to remove or reduce a white cloudiness degree.

19. An UV-blocking agent including the thin film of claim 11.

20. Cosmetics including the thin film of claim 11.
